# EUROPEAN PATENT APPLICATION

(11) **EP 4 193 956 A1**
(43) Date of publication of application: **14.06.2023**
(21) Application number: 21213670.9
(22) Date of filing: 10.12.2021
(51) Int. Cl.: A61B 90/00, A61B 34/20

(54) **OPTICAL TRACKING MARKERS**

(71) Applicant: Koninklijke Philips N.V., 5656 AG Eindhoven (NL)
(72) Inventor: TERMEER, Maurice Alain, Eindhoven (NL)
(74) Representative: Philips Intellectual Property & Standards

(57) **Abstract**

The present invention relates to optical marker tracking of medical devices. In order to provide optical tracking suitable for an increased variety of equipment and devices, a marker (10) for optical tracking of medical devices is provided. The marker comprises a pattern (12) with a plurality of at least a first type (14) and a second type (16) of optically identifiable segments. The optically identifiable segments of the first type have a first optical characteristic, and the optically identifiable segments of the second type have a second optical characteristic. The first optical characteristic and the second optical characteristic are distinctively different from each other for a designated optical camera-based tracking device. The segments are provided as quadrilaterals (18) arranged in a two-dimensionally alternating pattern (20). The quadrilaterals each comprise two first opposite sides (22) that are parallel two each other and two second opposite sides (24) that are non-parallel to each other. The first opposite sides of all of the quadrilaterals are arranged in an aligned manner defining at least three parallel primary grid-lines (28).

## Description

### FIELD OF THE INVENTION

The present invention relates to marker for optical tracking of medical devices and to optical tracking system for medical devices.

### BACKGROUND OF THE INVENTION

Optical tracking systems can be used during surgery, e.g. for navigation, tracking or also documenting purposes. Optical tracking systems have become commonplace in the field of medical industry. A navigation system can provide real-time information on the location of a surgical instrument relative to a subject. This can be useful, for example, during minimal invasive image guided interventions, which are increasingly replacing open surgery procedures. Optical tracking may use patterns that are detectable via optical cameras and which patterns allow a conclusion of the spatial orientation of a device to which the marker is attached. For example, interventional needles with a shaft diameter of only a few millimeter can be provided with a coded pattern that can be tracked by visible light cameras. It has been shown that an increasing need exists to also track devices larger than needles.

### SUMMARY OF THE INVENTION

There may thus be a need to provide optical tracking suitable for an increased variety of equipment and devices.

The object of the present invention is solved by the subject-matter of the independent claims; further embodiments are incorporated in the dependent claims. It should be noted that the following described aspects of the invention apply also for the marker for optical tracking of medical devices and for the optical tracking system for medical devices.

According to the present invention, a marker for optical tracking of medical devices is provided. The marker comprises a pattern with a plurality of at least a first type and a second type of optically identifiable segments. The optically identifiable segments of the first type have a first optical characteristic, and the optically identifiable segments of the second type have a second optical characteristic. The first optical characteristic and the second optical characteristic are distinctively different from each other for a designated optical camera-based tracking device. The segments are provided as quadrilaterals arranged in a two-dimensionally alternating pattern. The quadrilaterals each comprise two first opposite sides that are parallel to each other, and two second opposite sides that are non-parallel to each other. The first opposite sides of all of the quadrilaterals are arranged in an aligned manner defining at least three parallel primary grid-lines.

As an effect, a marker pattern is provided that allows an identification of a spatial orientation of the marker. The marker is suitable for mounting to larger diameters of devices.

According to an example, at least six parallel primary grid-lines are provided.

According to an example, the quadrilaterals are arranged with their vertices in a mutually meeting manner, wherein the edges of the quadrilaterals are forming a grid.

According to an example, the pattern is non-symmetrical in at least that pattern segments provide an unambiguous direction information in relation to a longitudinal extension direction of the marker.

According to an example, the pattern is arranged in a tubular manner with a longitudinal axis. The at least three parallel primary grid-lines are aligned with the longitudinal axis.

According to an example, the pattern is arranged in a semi-tubular manner with a longitudinal axis and an open cross-section. The at least three parallel primary grid-lines are aligned with the longitudinal axis.

As an option, it is provided to vary the diameter of the semi-tubular manner with the C-shaped cross section.

According to an example, the second opposite sides within the pattern of all of the quadrilaterals form at least one secondary gridline. Further, when arranged in a flat and unrolled way, the at least one secondary gridline is arranged transverse to the at least three parallel primary grid-lines.

As an option, the secondary gridline is provided as at least one of the group of a curved line, a wave-type line or a polygonal line with at least two segments.

As another option, the at least one secondary gridline is provided non-parallel to outer boundary lines of the pattern.

As an option, at cross points with one of the at least three parallel primary grid-lines, the at least one secondary gridline is non-perpendicular to the one of the at least three parallel primary grid-lines.

According to an example, a body structure is provided which is carrying the pattern. In an option, the body structure is i) a tube-shaped structure with the pattern provided on the outside of the tube-shaped structure. The tube axis forms a longitudinal axis of the marker. In another option, the body structure is ii) a singly curved open shell structure with the pattern provided on a convex outer side or a concave inner side of the shell structure.

The curvature axis forms a longitudinal axis of the marker.

It is also provided that the at least three parallel primary grid-lines are arranged aligned with the longitudinal axis of the marker.

According to an example, the pattern comprises at least one edge band provided along a boundary section that extends transverse to the primary grid-lines. The at least one edge band comprises one of the first optical characteristic and the second optical characteristic as the main optical characteristic.

The at least one edge band is arranged such that each of the primary grid-lines terminates in a part of the edge band with the main optical characteristic.

As an option, the pattern comprises a first edge band along the boundary section on one side of the pattern, and a second edge band along the boundary section on the other one side of the pattern, the first edge band having the first optical characteristic and the second edge band having the second optical characteristic.

According to the present invention, also an optical tracking system for medical devices is provided. The system comprises at least one marker and at least one optical camera-based tracking device. The at least one marker is provided as a marker for optical tracking of medical devices according to one of the preceding examples. The optical camera-based tracking device is configured to detect the first optical characteristic and the second optical characteristic and to provide image data for further image data processing steps.

The markers can be used, for example, for tracking devices and parts in an operation room in a hospital. A field of use is in augmented reality surgical navigation arrangements. The markers can be used, for example, during minimally invasive spine surgery for tracking bone probes, an awl, or a screwdriver while placing pedicle screws. The markers can be used for pedicle screws, beams and supports.

According to an aspect, a marker is provided that enables 6-DOF (degrees of freedom) optical tracking of instruments, e.g. with a diameter larger than 1 cm. The marker uses a checkered pattern wrapped around a cylinder, where the size of the quadrilaterals changes across the pattern to facilitate 6-DOF tracking.

According to an aspect, the base of the pattern is a checkered pattern. Checkered patterns are suitable for use in computer vision applications. According to an aspect, the pattern is aligned such that the lines formed by the pattern along one of its dimensions are parallel with a central axis of an instrument to be tracked. These lines remain straight under (perspective) projection from any angle. This makes it possible to use this new pattern for larger instruments.

For example, the basic shape of the marker is a cylinder. In an example, a regular cylinder is provided for sliding the marker over the tip of an instrument to attach it. In another example, the marker is provided as a partial cylinder for attaching from the side.

For example, the quadrilaterals vary in size across the marker, removing any rotational symmetries and thus enabling 6-DOF tracking. In case of a closed cylinder, the pattern is defined using two planes that are not perpendicular to the central axis of the cone, but at a slight angle. This gives a continuous variation around the marker. In case of an open cylinder, the pattern does not need to be continuous (in a circumferential sense). In an example, the size of the middle segments varies from smallest on one side of the opening to largest on the other side of the opening.

As an option, special bands are placed on the top and bottom of the marker with smaller quadrilaterals than the rest of the marker. The parts of these bands close to the straight lines of the pattern are all black on one side and all white on the other side of the marker. This is used to determine the direction of the marker and removes the symmetry across central axis of the cylinder.

As an option, in between the direction-encoding parts of the bands at the ends, a binary pattern is encoded using respective contrary black and white quadrilaterals. The binary pattern is an ID value that is encoded using error correction techniques and padding bits. As an option, this pattern is repeated three times around the marker, since typically only a part of the marker is visible to the tracking system. The pattern is constructed such that if at least one third of the pattern is visible, the original ID value can be reconstructed.

According to an aspect, a pattern is provided that comprises a checkered pattern with varyingly sized quadrilaterals, optionally a unique ID encoded on the ends of the marker, and further optionally a cylindrical shape, which can be a partial cylinder to aid in attaching the marker to an instrument. The pattern is provided for instruments with a diameter greater than approximately one centimeter.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows an example of a marker for optical tracking of medical devices in a perspective view as a closed cylinder.
Fig. 2 shows the marker design of Fig. 1 in an unfolded 2D view.
Fig. 3 shows an example of a marker in a perspective view as an open tubular shape, i.e. as an open cylinder.
Fig. 4 shows another example of a marker in a perspective view.
Fig. 5 shows the marker design of Fig. 4 in an unfolded 2D view.
Fig. 6 shows a further example of a marker in a perspective view.
Fig. 7 shows the marker design of Fig. 4 in an unfolded 2D view.
Fig. 8 shows a further example of a marker in an unfolded view.
Fig. 9 shows an example of an optical tracking system for medical devices.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

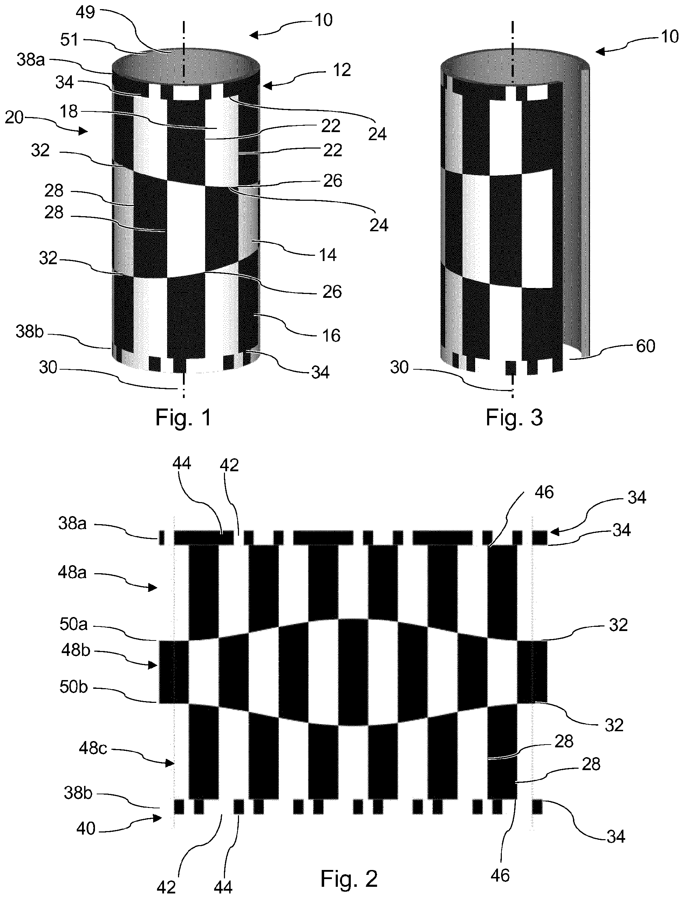

Fig. 1 shows an example of a marker 10 for optical tracking of medical devices in a perspective view. The marker 10 comprises a pattern 12 with a plurality of at least a first type 14 and a second type 16 of optically identifiable segments. The optically identifiable segments of the first type 14 have a first optical characteristic. The optically identifiable segments of the second type 16 have a second optical characteristic. The first optical characteristic and the second optical characteristic are distinctively different from each other for a designated optical camera-based tracking device. The segments are provided as quadrilaterals 18 arranged in a two-dimensionally alternating pattern 20. The quadrilaterals 18 each comprise two first opposite sides 22 that are parallel two each other and two second opposite sides 24 that are non-parallel to each other. The quadrilaterals 18 thus comprise four vertices 26 as corners. The first opposite sides 22 of all of the quadrilaterals 18 are arranged in an aligned manner defining at least three parallel primary grid-lines 28.

In the example of Fig. 1, the first optical characteristic, i.e. the first type 14 of optically identifiable segments, is white; and the second optical characteristic, i.e. the second type 16 of the optically identifiable segments, is black.

In another example, the first optical characteristic, i.e. the first type 14 of optically identifiable segments, is a first color; and the second optical characteristic, i.e. the second type 16 of the optically identifiable segments, is a second color.

The first opposite sides 22 and the second opposite sides 24 can also be referred to as first and second opposite edges.

In an example, at least six parallel primary grid-lines 28 are provided. For example, the six parallel primary grid-lines 28 are provided in 60° steps.

Having at least six parallel primary grid-lines 28 provides that even with one camera, the required minimum part of the pattern is visible. The pattern should have sufficient parallel primary grid-lines 28 such that in any orientation the camera system can triangulate at least three of those parallel primary grid-lines. How many lines the pattern should contain then of course depends on the camera system setup.

In an example, an even number of parallel primary grid-lines 28 is provided. This leads to a chess-board pattern when having a closed tubular shape. This provides the effect that when arranged around a tubular shaft, at least two of the radial blocks are visible, i.e. detectable for the camera.

In an example, the parallel primary grid-lines 28 are provided with a constant distance to each other, e.g. regularly displaced in a circumferential direction when arranged on a tubular shaft or the like.

In another example, the parallel primary grid-lines 28 are provided with a varying distance to each other, e.g. non-regularly displaced in the circumferential direction.

In an example, the camera system is provided such that the full 360 degrees of the marker is visible by at least two cameras. In such case, three parallel lines are sufficient.
In an example, the camera system is configured for a 120 degree view. Further, at least nine parallel primary grid-lines are provided.

In another example, 12 parallel primary grid-lines are provided to have some margin and to enable a facilitated and more robust way of the ID encoding.

As mentioned above, an even number is provided to avoid having two segments of the same color next to each other (at the matching boundary sections when wrapped around).

Shown as an option in Fig. 1, the quadrilaterals 18 are arranged with their vertices 26 in a mutually meeting manner, wherein the edges (first opposite sides 22) of the quadrilaterals are forming a grid.

Thus, the segments, also referred to as blocks, of same type touch each other only via the vertices 26, i.e. corners. In an example, segments of the same type do not have mutual second sides next to each other. In other words, adjacent segments with common side lines are of different type. A segment of the first type is arranged in an adjacent manner with segments of the second type and vice versa.

In an example, the vertices 26 of adjacent quadrilaterals 18 are mutually adjacent meeting in one point.

The term "pattern" relates to the repetitive arrangement of graphically distinct portions of a surface. The portions are arranged such that they follow a general concept, i.e. the concept of the pattern, but at least a part of the portions is distinct from other portions.

The term "optical characteristic" relates to the optical appearance of the portions. This comprises parameters like color or brightness but may also relate to roughness or reflective properties. In an example, the first and the second optical characteristics are provided as black and white (or at least a bright gray and a dark gray), or as complementary colors.

The term "optically identifiable" relates to properties that can be detected by optical means, like a camera. The term "optically" refers to the visible light spectrum, i.e. the light wave range visible by the human eye, but also refers to the infrared light wave range and the ultraviolet light wave range.

The term "camera-based tracking device" relates to devices with optical (visible, infrared, and ultraviolet light) sensors that are able to be used for tracking the pattern. Tracking refers to providing image data that can be used for image processing steps in which the pattern is detected for further analyzing steps.

In the context of the present application, the term "quadrilaterals" refers to segments of the marker pattern that comprise four corners and four connecting sides. In an example, the sides are straight lines, defining a quadrilateral in its geometrical meaning. In an example, one or two the sides are curved or polygon lines, i.e. non-straight lines, defining a quasi-quadrilateral in a wider sense, i.e. in its enlarged meaning as applied in the present situation. However, in the present context, the term quadrilateral relates to all different deviations as well as to the quadrilateral with four corners and four connecting linear sides. The term "quadrilaterals" thus refers to a polygon with four edges and four vertices. However, in the present context, the term "quadrilateral" relates to a geometrical form with a surface area that has at least four corners and four edges. In an example, exactly four corners are provided. In an option, all four edges of the quadrilateral are straight, i.e. linear, lines. In another option, the two sides that are parallel to each other are straight, i.e. linear, lines, whereas from the other two at least one is having a curved shape, i.e. nonlinear sides. In an example, at least one of the two non-parallel sides is providing a convex outer contour for the quadrilateral form. As an example, both non-parallel sides are providing a convex outer contour for the quadrilateral form. In another example, at least one of the two non-parallel sides is providing a concave outer contour for the quadrilateral form. As an example, both non-parallel sides are providing a concave outer contour for the quadrilateral form. As a further example, one of the non-parallel sides is providing a convex outer contour for the quadrilateral form and the other one of the non-parallel sides is providing a concave outer contour for the quadrilateral form.

Shown as an option in Fig. 1, the pattern 12 is arranged in a tubular manner with a longitudinal axis 30. The at least three parallel primary grid-lines 28 are aligned with the longitudinal axis 30.

In the example, the pattern 12 is arranged in a tubular manner with a closed cross-section in Fig. 1. For example, a closed cylinder is provided, for example with an O-shape cross section or a circular cross section.

In example, when the pattern is arranged in a tubular manner, the second opposite sides that are arranged within the pattern are defining at least one plane that is arranged in an inclined manner in relation to the longitudinal axis.

The term "within the pattern" relates to those opposite sides that are facing a further opposite side of an adjacent quadrilaterals, i.e. a next pattern segment.

The opposite sides that are not facing a pattern segment, but form an outer edge of the pattern, may also form a plane, e.g. transverse to the longitudinal axis, for example perpendicular to the longitudinal axis.

In an example, the pattern is arranged in a round tubular manner, for example with a circular or a non-circular, e.g. oval, or elliptic, cross-section.

In another example, the pattern is arranged in a rectangular tubular manner, for example with a rectangular or square cross-section.

In another example, the pattern is arranged in a polygonal manner, for example with a polygonal cross-section.

In a further example, the pattern is arranged in a circular tubular manner.

In an example, the second opposite sides that are arranged within the pattern are defining at least two planes, from which at least one is arranged in an inclined manner in relation to the longitudinal axis.

In another example, at least two planes are defined, from which one is arranged in an inclined manner in relation to the longitudinal axis and one in a perpendicular manner.

In a further example, at least two planes are defined, from which at least two are arranged in an inclined manner in relation to the longitudinal axis.

As an example, the two planes are arranged parallel.

As another example, the two planes are arranged inclined to each other.

In a further option (not shown in detail), the two sides that are parallel to each other are straight, i.e. linear, lines, whereas from the other two at least one is having a polygonal shape, i.e. a side comprising two or more linear line segments. In such a case, the "quadrilateral", as used in the present context, comprises four so-to-speak primary corners and one so-to-speak secondary corner.

The vertices, or corners, relate to points where two edges meet.

The two-dimensionally alternating pattern forms a sort of chess-board pattern, but with only one direction of the gridlines being straight and parallel lines. It is noted that the term "pattern" is used, referring to an arrangement of segments, from which at least a part is different from each other, in particular from neighboring segments, i.e. from adjacent segments. Hence, in the present context, the term pattern, usually referring to a repetition of same segments, is used for a structure that shows a concept more as a general order, but with distinctive differences of at least some of the segments. While neighboring segments are different from each other, they, i.e. their shape and size, might be repeated in the structure of the pattern in a displaced manner, i.e. with one or more segments between equal segments.

The term "optically identifiable segment" refers to the different surface areas of the pattern. The optically identifiable segments can also be referred to as the pattern segments or pattern elements.

The actual (minimum) size of the segments depends also on the resolution of the camera used for tracking. The minimum values of the aspect ratio are also given by the resolution of the camera.

Fig. 2 shows the marker design of Fig. 1 in an unfolded 2D (plan) view. In an example, the pattern is non-symmetrical in at least that pattern segments provide an unambiguous direction information in relation to a longitudinal extension direction of the marker.

As shown in Fig. 1, the second opposite sides 24 within the pattern of all of the quadrilaterals 18 form at least one secondary gridline 32. When arranged in a flat and unrolled way, the at least one secondary gridline 32 is arranged transverse to the at least three parallel primary grid-lines 28.

As an option, the secondary gridline 32 is provided as at least one of the group of a curved line, a wave-type line or a polygonal line with at least two segments.

As an option, the at least one secondary gridline 32 is provided non-parallel to outer boundary lines 34 of the pattern.

As an option, the at least one secondary gridline 32 is provided non-parallel to outer boundary lines 34 of the pattern.

As an option, at cross points with one of the at least three parallel primary grid-lines 28, the at least one secondary gridline 32 is non-perpendicular to the one of the at least three parallel primary grid-lines.

In an example, the at least one secondary gridline 32 is a having a shape of at least one of the group of a curve, a wave, or a polygonal line with at least two segments.

In an example, the at least one secondary gridline 32 comprises at least one of the group of a curve segment, a wave segment, and a linear segment.

Distinctively different comprises to differ in at least one parameter of the group of color and brightness value for the determined light wave spectrum of the optical camera-based tracking device.

In an example, the segments of the first type and the segments of the second type are distinctively detectable at least in one of the group of visible light wave range, infrared light wave range and ultraviolet light wave range.

In an example (see also Fig. 1 and Fig. 3), a body 51 structure is provided which is carrying the pattern. The body structure can be i) a tube-shaped structure 49 with the pattern provided on the outside of the tube-shaped structure. The tube axis forms the longitudinal axis 30 of the marker. The body structure can also be ii) a singly curved open shell structure 36 (see Fig. 3) with the pattern provided on a convex outer side or a concave inner side (not shown) of the shell structure 36. The curvature axis forms the longitudinal axis 30 of the marker. The at least three parallel primary grid-lines 28 are arranged aligned with the longitudinal axis 30 of the marker.

The term "arranged aligned with the longitudinal axis of the marker" refers to a parallel arrangement of the at least three parallel primary grid-lines 28 and the longitudinal axis 30 of the marker

As an example, the tubular manner comprises one of the group of: a cylinder with circular cross-section, a tube with round, non-circular cross-section, a prism, a frustrum and a truncated cone. The frustrum relates to a truncated structure with a polygonal cross-section. The truncated cone relates to a truncated structure with a circular cross-section. The pattern 12 is provided on such shapes.

Shown as an option in Fig. 1 and Fig. 2, the pattern 12 comprises at least one edge band 38 provided along a boundary section that extends transverse to the primary grid-lines 28. The at least one edge band 38 comprises one of the first optical characteristic and the second optical characteristic as the main optical characteristic. The at least one edge band 38 is arranged such that each of the primary grid-lines 28 terminates in a part of the edge band with the main optical characteristic.

In an option, shown in Fig. 2, the pattern comprises a first edge band 38a along the boundary section on one side of the pattern, and a second edge band 38b along the boundary section on the other one side of the pattern. The first edge band 38a is provided with the first optical characteristic and the second edge band 38b is provided with the second optical characteristic.

In another option, only one first edge band 38 is provided.

The term "main optical characteristic" relates to a structured edge band comprising several parts of different optical characteristics.

The "one side of the pattern" and the "other one side of the pattern" can also be referred to as the first boundary and the second boundary. The one side of the pattern and the other one side of the pattern refer to two opposing boundary sections, i.e. edge zones of the pattern, which are transverse, e.g. perpendicular, to the primary grid-lines.

The arrangement of a first and a second edge band allows a better distinction of the directions, by detecting in which optical characteristic, i.e. which color, the primary grid-lines are terminating, i.e. ending.

For example, a white (or bright) main optical characteristic is provided on the first boundary section indicating a "bottom" direction of the pattern (see Fig. 2) when arranged in the designated manner, e.g. of a device. For further example, a black (or dark) main optical characteristic is provided on the first boundary section indicating a "top" direction of the pattern when arranged in the designated manner, e.g. of a device.

The at least one edge band, for example the bottom edge band 38b, comprises an edge band pattern 40 comprising a plurality of first edge band segments 42 and second edge band segments 44 arranged in a coding scheme manner in order to provide encoded information by the at least one edge band. The first edge band segments 42 are having a first edge band optical characteristic and the second edge band segments 44 are having a second edge band optical characteristic that differs from the first edge band optical characteristic. As an option, the edge band pattern 40 is repeated at least three times along the edge.

In an option, only one of the at least one edge band comprises the edge band pattern 40.

In another option, two edge bands are provided and both comprise an example of the edge band pattern 40. For example, both edge bands can comprise the same edge band pattern.

In another option, two edge bands are provided (see Fig. 2) and both comprise an example of the edge band pattern 40. For example, both edge bands comprise a different edge band pattern. As shown in Fig. 3, the upper edge band comprises an edge band pattern 40 comprising a plurality of the first edge band segments 42 and the second edge band segments 44 arranged in a coding scheme manner in order to provide encoded information by the upper edge band. The first edge band segments 42 of the upper edge band are having a first edge band optical characteristic and the second edge band segments 44 of the upper edge band are having a second edge band optical characteristic that differs from the first edge band optical characteristic.

In an option, the first edge band optical characteristic of the upper edge band is opposite to the first edge band optical characteristic of the lower edge band. Similar, in an option, the second edge band optical characteristic of the upper edge band is opposite to the second edge band optical characteristic of the lower edge band.

In another option, the first edge band optical characteristic of the upper edge band is the same as the first edge band optical characteristic of the lower edge band. The second edge band optical characteristic of the upper edge band is the same as the second edge band optical characteristic of the lower edge band.

As an option, the second edge band pattern 40 is also repeated at least three times along the edge.

The edge band pattern/s is/are arranged such that a sequence of the first and second edge band segments is provided with alternating first and second edge band optical characteristics. In an example, the edge band segments, extending across the complete width of the edge band, i.e. in the direction of the primary grid-lines, have varying lengths in a circumferential direction. However, the transition lines from the first edge band optical characteristic to the second edge band optical characteristic and vice versa is arranged offset to the primary grid-lines to ensure that each primary gridline 28 ends at an end or terminating point 46 in a field of either the first edge band optical characteristic (for one of the edge bands) or the second edge band optical characteristic (for the other one of the edge bands).

In an example, the first edge band optical characteristic is provided similar to the first optical characteristic of the (main) pattern. As an option, in addition or alternatively, the second edge band optical characteristic is provided similar to the second optical characteristic of the (main) pattern.

For example, the first edge band optical characteristic is the same as the first optical characteristic of the (main) pattern. As an option, in addition or alternatively, the second edge band optical characteristic is the same as the second optical characteristic of the (main) pattern.

To repeat the edge band pattern at least three times along the edge provides the advantage that the encoded information can be detected without the need to cover the complete edge band pattern. When the pattern is provided in a tubular shape, the edge band pattern is visible in one view when the edge band pattern is arranged over maximum 120° of the circumferential direction (see also above discussing the number of grid-lines).

As an example, the edge band pattern provides encoded information, for example referring to the marked tool or device comes from.

To apply two edge bands results in the possibility of being able to provide more data in combination.

In an example, the bands as described are combined with a pattern that encodes an ID. The segments that make up the pattern are shifted by half their width compared to the parallel lines of the main pattern. The segments in line with those parallel lines form the band. The other segments form the binary signal encoding some ID.

In an option, error-correction techniques are provided to make sure the correct ID is recovered if some of the segments are not read correctly, i.e. misread.

In an example, a tracking system is provided that comprises two or more cameras placed close together looking in approximately the same direction.

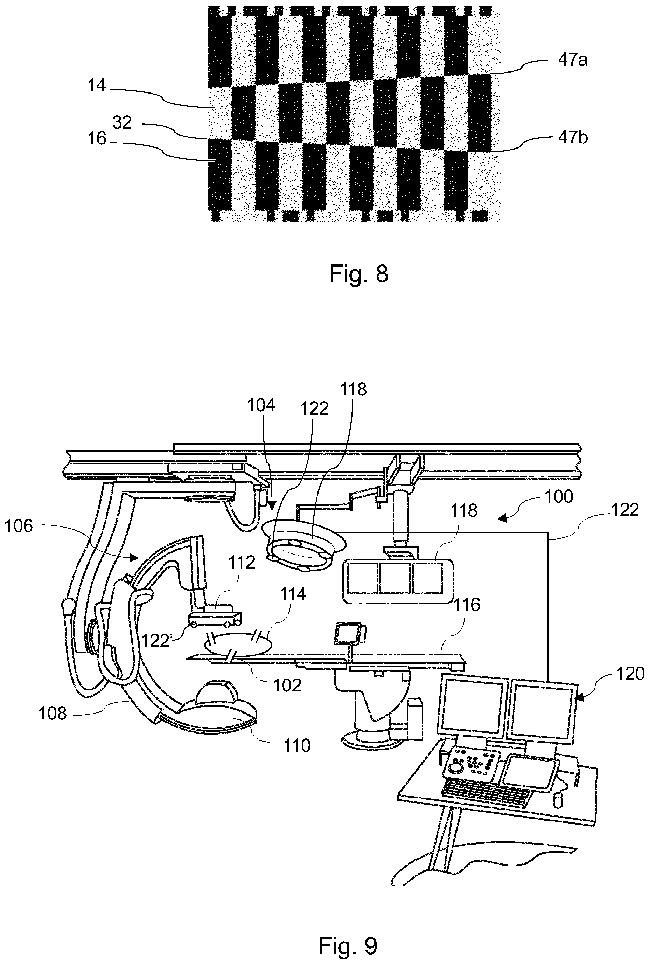

Fig. 2 shows a pattern with a plurality of different horizontal (relating to the drawing in its portrait orientation of the page) zones, such as an upper zone 48a, a central zone 48b and a lower zone 48c. A first boundary line 50a is formed by the respective second opposite sides 24. The first boundary line 50a is shown with a wave-like contour in Fig. 2 as an option. Further, a second boundary line 50b is formed by the respective second opposite sides 24. The second boundary line 50b is shown with a wave-like contour in Fig. 2 as an option. The contours may be non-parallel, for example with a contrary increase or decrease ratio over time. In another example, see also Fig. 8, one or all of the second boundary lines 32 are provided as a straight lines 47a, 47b when the pattern is arranged unrolled.

Fig. 3 shows an example of the marker 10 in a perspective view as an open tubular shape, i.e. as an open cylinder.

In another example, shown as another option in Fig. 3, the marker 10 is provided as an open cylinder, for example with a longitudinal gap 60. The pattern 12 is arranged in a tubular manner with an open cross-section, for example with a C-shape cross section.

In an example, the pattern 12 is arranged in a semi-tubular manner with a longitudinal axis and an open cross-section. The at least three parallel primary grid-lines are aligned with the longitudinal axis.

As an option, the diameter of the semi-tubular manner with the C-shaped cross section is varied. The pattern 12 is provided with an intersecting line from one side of the C to another side of the C.

In an example, one or more cone-shaped marker is provided as an "open cylinder", i.e. a cone-shaped form, but with an axially oriented slit or gap, i.e. a non-continuous surface in circumferential direction.

In another example, one or more cone-shaped marker is provided as a "closed cylinder", i.e. a cone-shaped form with a continuous surface in circumferential direction, i.e. without an axially oriented slit or gap.

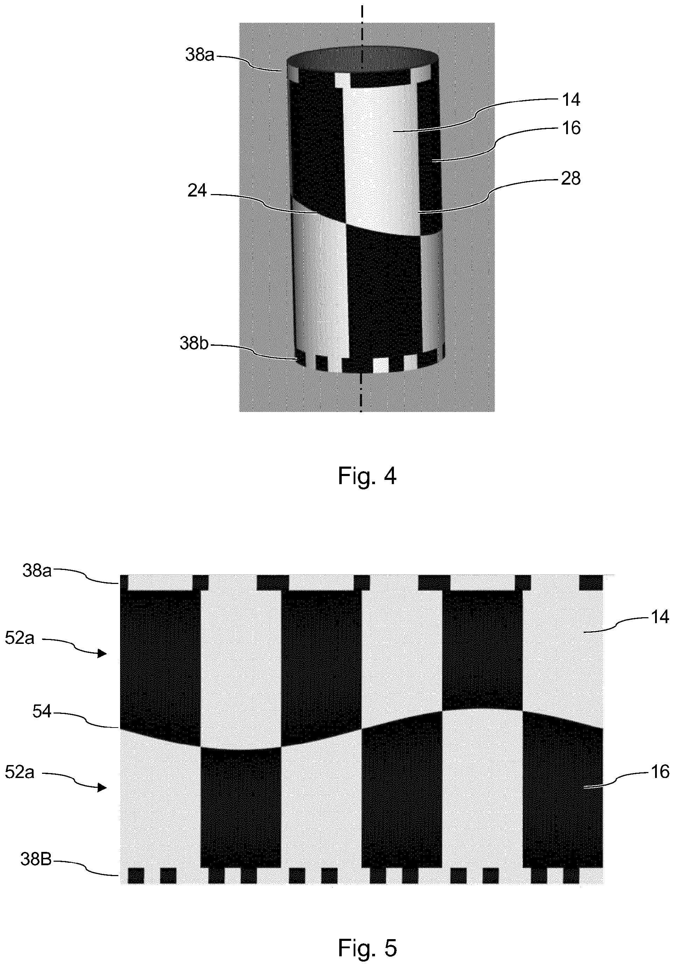

Fig. 4 shows another example of a marker in a perspective view. Fig. 5 shows the marker design of Fig. 4 in an unfolded 2D (plan) view.

Fig. 5 shows a pattern with a plurality of different horizontal (relating to the drawing in its portrait orientation of the page) zones, such as an upper zone 52a and a lower zone 52b. A boundary line 54 is formed by the respective second opposite sides 24. The boundary line 54a is shown with a wave-like contour in Fig. 5 as an option.

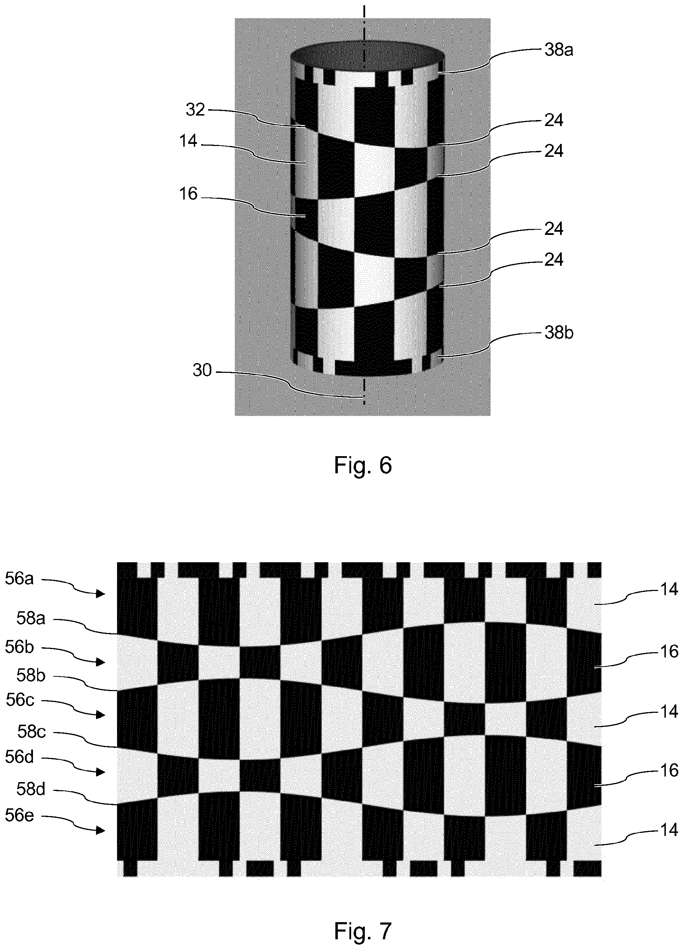

Fig. 6 shows a further example of a marker in a perspective view. Fig. 7 shows the marker design of Fig. 4 in an unfolded 2D (plan) view.

Fig. 7 shows a pattern with a plurality of different horizontal (relating to the drawing in its portrait orientation of the page) zones, such as a first zone 56a, a second zone 56b, a third zone 56c, a fourth zone 56d and a fifth zone 56e.

A first boundary line 50a is formed by the respective second opposite sides 24. The first boundary line 58a is shown with a wave-like contour in Fig. 2 as an option. A second boundary line 58b is formed by the respective second opposite sides 24. The second boundary line 58b is shown with a wave-like contour in Fig. 2 as an option. A third boundary line 58c is formed by the respective second opposite sides 24. The third boundary line 58a is shown with a wave-like contour in Fig. 2 as an option. A fourth boundary line 58d is formed by the respective second opposite sides 24. The fourth boundary line 58d is shown with a wave-like contour in Fig. 2 as an option.

The contours (of the boundary lines) may be non-parallel, for example with a contrary increasing or decreasing ratio over time.

Fig. 8 shows an example where the pattern is provided with an intersecting line (shown in form of the straight lines 47a, 47b) from one side of the C to another side of the C. Shown as an option in Fig. 8, the pattern is provided with two or more intersecting lines from one side of the C to another side of the C.

In an example, the pattern as shown in Fig. 8 is applied for an open cylinder or open cone-shaped form, thus having the C-shaped cross section.

In another example, the pattern as shown in Fig. 8 is applied for a closed cylinder or closed cone-shaped form.

Fig. 9 shows an example of an optical tracking system 100 for medical devices. The optical tracking system 100 comprises at least one marker 102 and at least one optical camera-based tracking device 104. The at least one marker 102 is provided as an example of the marker 10 for optical tracking of medical devices according to one of the preceding examples. The optical camera-based tracking device 104 is configured to detect the first optical characteristic and the second optical characteristic and to provide image data for further image data processing steps. The optical tracking system 100 is depicted in an environment of an operation room in a hospital. For example, an X-ray imaging system 106 is provided as a movably mounted C-arm 108. An X-ray source 110 and an X-ray detector 112 are provided at opposite ends of the C-arm 108. An object 114 is arranged on a subject support 116. The markers 102 are attached to the object 114. Further, a monitor arrangement 118 is indicated and a console 120 in the right lower foreground. The optical camera-based tracking device 104 comprises a support structure 118 to which four cameras 122 are mounted. A line 122 indicates the data connection of the optical camera-based tracking device 104 to the console 120.

In another option, the optical camera-based tracking device 104, i.e. the camera system, is embedded in the X-ray detector 112. For example, three or more cameras 122' are provided in an integrated manner with the housing of the detector 112, as indicated with hashed lines in Fig. 9.

The term "subject" may also be referred to as individual. The "subject" may further also be referred to as patient, although it is noted that this term does not indicate whether any illness or disease is actually present with the subject.

It has to be noted that embodiments of the invention are described with reference to different subject matters. In particular, some embodiments are described with reference to device type claims whereas other embodiments are described with reference to the system type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A marker (10) for optical tracking of medical devices, the marker comprising:
- a pattern (12) with a plurality of at least a first type (14) and a second type (16) of optically identifiable segments;
wherein the optically identifiable segments of the first type have a first optical characteristic, and the optically identifiable segments of the second type have a second optical characteristic;
wherein the first optical characteristic and the second optical characteristic are distinctively different from each other for a designated optical camera-based tracking device;
wherein the segments are provided as quadrilaterals (18) arranged in a two-dimensionally alternating pattern (20);
wherein the quadrilaterals each comprise two first opposite sides (22) that are parallel two each other and two second opposite sides (24) that are non-parallel to each other; and
wherein the first opposite sides of all of the quadrilaterals are arranged in an aligned manner defining at least three parallel primary grid-lines (28).

2. Marker according to claim 1, wherein at least six parallel primary grid-lines are provided.

3. Marker according to claim 1 or 2, wherein the quadrilaterals are arranged with their vertices (26) in a mutually meeting manner, wherein the edges of the quadrilaterals are forming a grid.

4. Marker according to one of the preceding claims, wherein the pattern is non-symmetrical in at least that pattern segments provide an unambiguous direction information in relation to a longitudinal extension direction of the marker.

5. Marker according to one of the preceding claims, wherein the pattern is arranged in a tubular manner with a longitudinal axis (30); and
wherein the at least three parallel primary grid-lines are aligned with the longitudinal axis.

6. Marker according to one of the preceding claims, wherein, when the pattern is arranged in a tubular manner, the second opposite sides that are arranged within the pattern are defining at least one plane that is arranged in an inclined manner in relation to the longitudinal axis.

7. Marker according to one of the preceding claims, wherein the pattern is arranged in a semi-tubular manner with a longitudinal axis and an open cross-section;
wherein the at least three parallel primary grid-lines are aligned with the longitudinal axis; and
wherein, preferably, to vary the diameter of the semi-tubular manner with the C-shaped cross section, the pattern is provided with an intersecting line from one side of the C to another side of the C.

8. Marker according to one of the preceding claims, wherein the second opposite sides within the pattern of all of the quadrilaterals form at least one secondary gridline (32); and
wherein, when arranged in a flat and unrolled way:
- the at least one secondary gridline is arranged transverse to the at least three parallel primary grid-lines;
- preferably, the secondary gridline is provided as at least one of the group of a curved line, a wave-type line or a polygonal line with at least two segments;
- preferably, the at least one secondary gridline is provided non-parallel to outer boundary lines of the pattern; and
- preferably, at cross points with one of the at least three parallel primary grid-lines, the at least one secondary gridline is non-perpendicular to the one of the at least three parallel primary grid-lines.

9. Marker according to one of the preceding claims, wherein distinctively different comprises to differ in at least one parameter of the group of color and brightness value for the determined light wave spectrum of the optical camera-based tracking device.

10. Marker according to one of the preceding claims, wherein the segments of the first type and the segments of the second type are distinctively detectable at least in one of the group of visible light wave range, infrared light wave range and ultraviolet light wave range.

11. Marker according to one of the preceding claims, wherein a body structure (51) is provided which is carrying the pattern;
wherein the body structure is:
i) a tube-shaped structure (49) with the pattern provided on the outside of the tube-shaped structure; wherein the tube axis forms a longitudinal axis of the marker; or
ii) a singly curved open shell structure (36) with the pattern provided on a convex outer side or a concave inner side of the shell structure; wherein the curvature axis forms a longitudinal axis of the marker; and
wherein the at least three parallel primary grid-lines are arranged aligned with the longitudinal axis of the marker.

12. Marker according to one of the preceding claims, wherein the tubular manner comprises one of the group of:
- a cylinder with circular cross-section;
- a tube with round, non-circular cross-section;
- a prism;
- a frustrum; and
- a truncated cone.

13. Marker according to one of the preceding claims, wherein the pattern comprises at least one edge band (38) provided along a boundary section that extends transverse to the primary grid-lines;
wherein the at least one edge band comprises one of the first optical characteristic and the second optical characteristic as the main optical characteristic;
wherein the at least one edge band is arranged such that each of the primary grid-lines terminates in a part of the edge band with the main optical characteristic; and
wherein, preferably, the pattern comprises a first edge band (38a) along the boundary section on one side of the pattern, and a second edge band (38b) along the boundary section on the other one side of the pattern, the first edge band having the first optical characteristic and the second edge band having the second optical characteristic.

14. Marker according to claim 12, wherein the at least one edge band comprises an edge band pattern (40) comprising a plurality of first and second edge band segments (42, 44) arranged in a coding scheme manner in order to provide encoded information by the at least one edge band;
wherein the first edge band segments are having a first edge band optical characteristic and the second edge band segments are having a second edge band optical characteristic that differs from the first edge band optical characteristic;
wherein, preferably, the edge band pattern is repeated at least three times along the edge.

15. An optical tracking system (100) for medical devices, comprising:
- at least one marker (10); and
- at least one optical camera-based tracking device (102);
wherein the at least one marker is provided as a marker for optical tracking of medical devices according to one of the preceding claims; and
wherein the optical camera-based tracking device is configured to detect the first optical characteristic and the second optical characteristic and to provide image data for further image data processing steps.
